# EUROPEAN PATENT SPECIFICATION

(11) **EP 0 717 838 B1**
(45) Date of publication and mention of the grant of the patent: **12.05.2004**
(21) Application number: 94926662.1
(22) Date of filing: 31.08.1994
(51) Int. Cl.: G01N 1/12, B01L 3/00, C12M 1/14, G01N 33/49, G01N 33/86, C12Q 1/56

(54) **REAGENT AND METHOD OF ITS USE**
REAGENZ UND VERFAHREN FÜR SEINE ANWENDUNG
REACTIF ET PROCEDE D'UTILISATION DUDIT REACTIF

(30) Priority: 31.08.1993 US 114579
(43) Date of publication of application: 26.06.1996
(73) Proprietor: Roche Diagnostics Corporation, Indianapolis, IN 46250 (US)
(72) Inventor: ALDERINK, Michael, W., Westfield, IN 46074 (US); FISHER, Paul, R., Fishers, IN 46038 (US); GRAGE, Henry, M., Jr., Carmel, IN 46032 (US); MISHRA, Sarmishtha, M., Indianapolis, IN 46250 (US)
(74) Representative: Jung, Michael, Dr.
(86) International application number: PCT/US1994/009889
(87) International publication number: WO 1995/006868

(56) References cited:
- EP-A- 0 176 638
- US-A- 4 452 773
- US-A- 4 755 461
- US-A- 4 849 340
- US-A- 5 102 788
- US-A- 5 110 727
- US-A- 5 240 843

## Description

### Background Of The Invention

### 1. Field of the Invention:

The present invention relates to new reagent compositions for use in coagulation or clotting time assays.

### 2. Prior Art:

Coagulation assays have gained acceptance as an important tool in the management of patients being treated with anticoagulants. In such assays a specimen of whole blood or plasma is assayed for clotting time or coagulation time as an indication of the patient's response to a particular dosage of anticoagulant.

Coagulation or clotting time assays are also often performed on blood or plasma from a patient prior to a surgical procedure in order to provide medical professionals with information about the clotting time of the blood of a patient who is not necessarily being treated with anticoagulants.

Industry has directed much effort to designing and commercializing coagulation time assays and assay devices which are relatively simple to use and relatively inexpensive to buy and to operate. Much attention has been given to assay devices which use disposable slides or cartridges. The slides or cartridges usually contain reagents for performance of the assay and also provide a convenient location for reacting a specimen with the reagents and for monitoring the resulting reaction as a part of the assay.

One such coagulation time assay device monitors changes in the flow rate of particles in a specimen as an indication of coagulation time. In such assay devices a blood or plasma sample is moved by capillary action through a pathway in a cartridge where it is mixed with a dry coagulation reagent containing thromboplastin and is then monitored to observe changes in flow rate. The flow rate changes may, for example, be monitored by observing the passage of particles, such as blood cells, past a beam of collimated light which is directed across the passageway in the cartridge.

Such assays and assay devices are described in, for example, U.S. Patents Number 5,140,161, 4,963,498, 4,756,884, 5,164,598, 5,204,525 and 5144,139 and in European Patent Publications No. 0 397 424 A2 and 0 394 070 A1.

A different type of coagulation or clotting time assay uses capillary forces in a passageway to draw a liquid blood or plasma specimen to a reaction site where it is held. The specimen solubilizes a dry reagent which is also in the capillary passageway and/or at the reaction site. The reagent contains thromboplastin, which initiates clotting of the specimen, and magnetizable particles. The particles are oscillated in the liquid at the reaction site by toggling between external magnetic fields which are oriented at an angle to each other to cause an oscillating magnetic field which affects the particles. As clot formation progresses, particle oscillation is hindered. Changes in the oscillation of the particles provide a detectable signal which can be correlated to clotting time of the specimen.

Such assays, which are hereinafter for convenience referred to as oscillating particle assays, and cartridges for use in such assays are described, for example, in U.S. Patents No. 4,849,340, and 5,110,727 and in PCT International Publications No. WO 92/01065 and WO 89/10788. U.S. 5,110,727 describes air drying a thromboplastin reagent combined with magnetizable particles in a capillary space, but indicates that freeze drying is preferred.

Efforts to commercialize coagulation assay devices using cartridges having capillary spaces and including particulate indicator reagents have normally included freeze drying at least some of the reagents in the desired locations in the capillary spaces. Freeze drying has been preferred in the past because the heat of air drying tends to denature the thromboplastin reagent, requiring the use of large amounts during the manufacturing process in order to obtain assay devices with a useful amount of active reagent remaining and because freeze dried materials are readily resolubilized during the assay. These efforts have been technically successful, although freeze drying has been found to be relatively slow, complex and expensive, lending itself to batch processing rather than to high throughput continuous processing, thus reducing the commercial attractiveness of such assays. There has been a need for an innovation which avoids the undesirable complications and relatively high expense of freeze drying and which makes air drying of reagents for use in oscillating particle coagulation assays more attractive.

### Summary of the Invention

It is an object of the present invention to overcome the disadvantages of the prior art, especially freeze drying of reagents in a capillary space during manufacturing.

It is also an object of the present invention to produce an air dryable reagent solution and to form it into a stable, dry reagent coating on a reagent device surface.

It is a further object of the present invention to provide a coated, air dried reagent composition which simulates the solubility of freeze dried reagents.

It is still another object of the present invention to manufacture coagulation assay devices by a continuous process.

It is also an object of the present invention to make a carrier for reagents used in assays which is coatable and which is readily solubilized.

These and other objects are provided by the present invention.

The present invention is based on the discovery that air dryable, readily soluble reagent compositions which are dryable as coatings on commonly used materials, such as plastics and which are as readily resolubilized as freeze dried materials can be formed by combining the active reagent components with a novel carrier mixture of soluble carbohydrates of higher and lower molecular weights. The higher molecular weight carbohydrates in the carrier are selected to lend coating properties to the composition, to resist aggregating of reagent particles in the solution during manufacture and to optimize resolubilization characteristics of the reagent layer during assay performance. The lower molecular weight carbohydrate in the carrier is selected to increase the solubility of the dried reagent composition when contacted with a liquid specimen.

The present invention is also based on the discovery that a system of such reagents which is applied as separate layers in the capillary space of an oscillating particle reagent device can avoid the manufacturing problems of the past related to the loss of thromboplastin activity during heat drying and the corresponding need to use a freeze drying step and to the batch processing inherent in freeze drying.

In one aspect, the present invention is the reagent composition.

In yet another aspect, the invention is a system of reagent compositions.

### BRIEF DESCRIPTION OF THE DRAWINGS

The present invention is described below in greater detail and with reference to the drawings.
FIG. 1 shows in schematic perspective view an assay device according to the present invention which is useful in performing clotting time assays.
FIG. 2 shows in schematic cross section a preferred embodiment the device of FIG. 1 along line 2-2.
FIG. 3 shows in schematic cross section another embodiment of a capillary device according to the present invention.

### DETAILED DESCRIPTION

This invention provides a carrier material for reagents. The reagents may be those which are useful in assays. However, the carrier materials of this invention will have wide applicability wherever it is desirable to have a coatable carrier which is readily resoluble.

This invention also provides reagent compositions which are useful in coagulation (clotting time) assays. These compositions are particularly useful in assay devices in which a liquid blood or plasma specimen is transported by capillary action into a cartridge which contains the reagent and in which the assay is performed, although the reagent compositions are also useful for the performance of coagulation assays in cuvettes, on slides and in laboratory dishes.

Reagent compositions of this invention may be formulated to be useful in both the flow rate change assays and the oscillating magnetizable particle assays described above, although they have been developed primarily for use in the oscillating magnetizable particle type of assay. It will be immediately apparent to those of ordinary skill in the arts of assays and of assay devices that the present invention applies to assays and assay devices other than those used to determine clotting time. The present invention can be used in any assay in which a reagent containing composition is coated on a surface by drying and later brought back into a solution by contacting with a test specimen. Such other embodiments are intended to be within the scope of the appended claims, although the following description focuses on the use of the invention in coagulation assays of the oscillating particle type.

These compositions are first formed as aqueous solutions which can be air dried in place during manufacturing of assay devices. In one such solution, the thromboplastin and indicator reagents (magnetizable particles) used in coagulation assays are mixed in aqueous solution with a carrier of soluble carbohydrate material having both higher and lower molecular weights. The carbohydrate material is selected to have a higher molecular weight portion to provide coating characteristics to the solution as it dries. Also, when the reagent comprises particulate material, such as magnetizable particles, the high molecular weight carbohydrate fraction is selected to prevent the particulate material from aggregating during the manufacturing process.

The lower molecular weight fraction is selected to improve the ability of the carrier to be solubilized by a liquid specimen but not to overwhelm the ability of the higher molecular weight fraction to prevent such aggregation

Many combinations of high and low molecular weight carbohydrates will be useful as the carrier in this invention. Good results have been obtained when the carbohydrate mixture is a mixture of saccharides. In one especially useful embodiment the high molecular weight carbohydrates are provided by agglomerated corn syrup solids while the low molecular weight carbohydrates are provided by disaccharides, such as sucrose. The preferred agglomerated corn syrup solids have a carbohydrate profile of: monosaccharides 2.3%, disaccharides 7.5%, trisaccharides 9.1%, tetrasaccharides 6.8% and pentasaccharides and above (saccharides greater than pentasaccharides) 74.4%. Preferably, the disaccharides and the agglomerated corn syrup solids are added to an aqueous reagent solution in amounts of about 4 grams each per 100 grams of total solution.

The weight ratio of disaccharides to higher molecular weight carbohydrates in the carrier can vary. If there is too little disaccharide, the reagent layer will not readily go into solution when contacted by a liquid specimen. If there is too great a proportion of disaccharide, the solution will not dry to a proper coating and the magnetizable particles may agglutinate.

A preferred weight ratio of disaccharide to pentasaccharide and above has been determined by experimentation to be about 1:0.75 and can be obtained by mixing in aqueous solution substantially equal parts of sucrose and commercially available agglutinated corn syrup solids.

The soluble carbohydrate materials having the desired mixture of molecular weights will provide a carrier material which can be coated on surfaces and which will readily resolubilize upon contact with a liquid specimen such as blood or plasma. The carrier material can be mixed with reagents to obtain reagent compositions.

Reagent compositions according to the present invention are made by mixing the active reagent ingredients in an aqueous solution of the carrier and drying.

Experimental work has shown that the range of both disaccharides and agglomerated corn syrup solids can vary somewhat from the preferred embodiment and still produce solutions which can be dried down to useful reagent compositions. For example, disaccharide can be added to an aqueous solution which also contains thromboplastin and/or magnetizable particles in a range of from about 2 to about 6 g. per 100 g. of total solution or suspension. Concentrations of disaccharides of less than about 2 g. per 100 g. of total solution or total suspension result in reagent layers which are undesirably slow to solubilize upon incubation with a liquid specimen while concentrations of greater than about 6 g. per 100 g. of total solution produce reagent compositions which begin to lose their coating characteristics.

The higher molecular weight saccharides (agglomerated corn syrup solids) can be added to an aqueous solution in a concentration range of from about 1 to about 6 g. per 100 g. of total solution and still produce useful coatings of reagent composition. However, concentrations below about 1 g. per 100 g. of total solution will result in reagent layers which lack the desired coating characteristics, and concentrations above about 6 g. per 100 g. of total solution will result in reagent composition coatings which are undesirably slow to go back into solution when contacted with a liquid specimen.

The corn syrup solids used in the preferred embodiment are available from Grain Processing Corporation, Muscatine, Iowa, and are sold under the trade name Maltrin QD(tm) M600. The disaccharides are preferably sucrose.

When reagents are being made for the oscillating magnetizable particle type of coagulation assays, magnetizable particles are added to the aqueous solution in an amount sufficient to give a signal which can be detected in response to the oscillating magnetic field during the performance of an assay. The concentration range of magnetizable particles which is useful has been found to range from about 0.1 to about 2.0 g. per 100 g. of total materials before the suspension is coated onto a device surface and dried.

Magnetizable particle concentrations of less than about 0.1 g. per 100 g. of total suspension tend not to be present in the final reagent layer in sufficient density to give a detectable signal. Although experimental work has demonstrated that about 2.0 g. of magnetizable particles per 100 g. of total suspension will produce reagent layers which will function correctly in a coagulation assay, no improvement in signal has been noted for concentrations in excess of 0.6 g. per 100 g. of water, making 0.6 g. the preferred concentration.

As is described in U.S. Patent 5,110,727, a variety of magnetizable particles will work in the oscillating magnetizable particle type of coagulation assay. A preferred particle for use in the present invention is Fe₃O₄ having a particle size of about 0.3 micron. Other particles useful in the present invention are described in that publication, which is available to the skilled artisan, and will not be repeated here.

The thromboplastin reagent may be present in the reagent composition or in the system of reagent compositions in any concentration which is sufficient to initiate a clotting cascade in the specimen. Typically this will be from about 4.8 to about 5.0 mg (measured as total protien)/ml of the aqueous solution before drying, although useful results have been obtained with reagent compositions dried from solutions having as little as 3.2 mg/ml and as great as 6 mg/ml. (Wherever concentrations of thromboplastin or ratios of thromboplastin and other elements are mentioned in the specification and in the appended claims, it is understood that thromboplastin as total protien is intended).

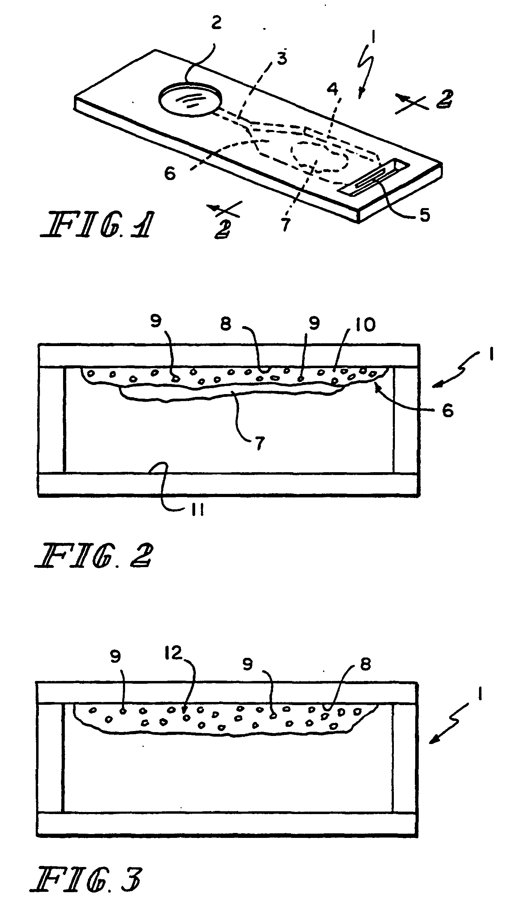

The concentrations of the components in the dried reagent layer may vary considerably and still be within the scope of the invention. It has been determined by experimental data that, when all of the components of a coagulation assay are contained in a single reagent composition, as is shown in FIG. 3, the preferred weight ratio of sucrose : agglomerated corn syrup solids : magnetizable particles : thromboplastin is 120 : 120 : 18 : 1. However useful compositions can include sucrose and agglomerated corn syrup solids weight ratios of from about 105 to about 160; magnetizable particle weight ratios of from about 12 to about 24, and thromboplastin weight ratios of from about 0.64 to about 1.2. These ratios relate to the relative concentrations by weight of the reagents in the solutions described above and to the reasons for the useful ranges also mentioned above.

In a preferred embodiment such as that shown in FIG. 2 the reagents necessary for a coagulation assay are provided in the capillary space of an oscillating particle device as a system of separate layers as is shown in FIG. 2. In such an embodiment the magnetizable particles are held in a first reagent layer 6 which normally covers substantially all of one surface of the capillary space while the thromboplastin reagent is held in a second reagent layer 7 which normally covers only the site at which a coagulation reaction will be monitored.

In such a preferred embodiment the magnetizable particle reagent layer 6 will have a concentration of particles, low molecular weight carbohydrates and high molecular weight carbohydrates substantially similar to that described above for a single layer reagent composition. The preferred concentration of these components in the reagent solution before drying into a reagent composition is about 0.6g particles for each 100 g. of total suspension and about 4 g. each of disaccharide and pentasaccharide and above for each 100 g. of water. In the dried reagent composition the weight ratio of sucrose : agglomerated corn syrup solids and magnetizable particles is 6.66 : 6.66 : 1. Experimental data has shown that useful results can be expected when the sucrose and agglomerated corn syrup solids weight ratios range from about 6 to about 9 while the magnetizable particles weight ratio ranges from about .66 to about 1.33.

The second part of the system of reagent layers in the preferred embodiment is the thromboplastin reagent layer 7. It can be coated on a separate surface of the interior of the capillary space in the assay device or it can be coated at a selected location on the first reagent layer.

As described above, the thromboplastin may be present in any concentration which will initiate a coagulation reaction useful in the assay. Experimental analysis has shown the preferred concentration (measured as total protien) in the solution used to form the second layer is in the range of from about 4.8 to about 5.0 mg/ml of reagent solution. This concentration is achieved by mixing about 100ml of a thromboplastin-containing extract of rabbit brain powder with about 2.96g each of disaccharide and pentasaccharide and above, as described above, to form a reagent solution and then air drying the solution in place.

The preferred weight ratio of sucrose : agglutinated corn syrup solids : thromboplastin (total protien) in the second layer has been determined to be 6 : 6 : 1. A useful range of weight ratios, however, has been determined by experimental data to be from about 4 to about 9 for the sucrose and the agglutinated corn syrup solids and from about 0.64 to about 1.2 for the thromboplastin.

The ranges of useful weight ratios of components in both layers correspond to the useful ranges of components in the solutions described above and to the reasons for the limits to those ranges.

It will be readily apparent to the skilled artisan that film formers, processing aids and other such ingredients can be included in the reagent compositions of the present invention as long as they don't interfere with the performance of the reagent in an assay.

Referring more specifically to the drawings, FIG. 1 shows an assay device **1** which is useful in the oscillating particle type of coagulation assay which is described more fully in U.S. Patents No. 4,849,340, and 5,110,727, mentioned above. A liquid specimen such as blood or plasma is applied to sample port **1** where it is drawn by capillary forces into capillary passageway **3** and moved to assay site **4**, which is also a capillary space. Assay site **4** is the location at which the oscillation of magnetizable particles will be monitored during the assay.

Capillary passageway **3** and assay site **4** are internal to device **1** and are indicated by dotted lines. Vent port **5** is open to the atmosphere and prevents a buildup of air pressure from acting against capillary flow of a liquid reagent in the capillary passageway.

One internal surface of the capillary space is coated with first reagent layer **6**. First reagent layer **6** includes magnetizable particles which have been dispersed in an aqueous solution of carbohydrates of both higher and lower molecular weights and then air dried in place in capillary spaces **3** and **4** of device **1**. First reagent layer **6** is described in greater detail above.

Second reagent layer **7** is placed on first reagent layer **6** substantially at assay site **4**. Reagent layer **7** is made by adding a mixture of carbohydrates of both higher and lower molecular weights to an aqueous thromboplastin reagent to form a solution and then air drying the solution in place. A more detailed description of reagent layer **7** is above.

The construction shown in FIG. 2 is a preferred embodiment. It will be readily apparent to skilled artisans that first reagent layer **6** could be positioned on any surface of the interior of capillary spaces **3** and **4** as long as magnetizable particles are provided to reaction site **4** when layer **6** is resolubilized by a liquid specimen. Likewise, second reagent layer **7** can also be anywhere within capillary spaces **3** and **4**, although it is less desirable to position layer **7** in capillary space **3** so as to discourage the start of coagulation before it can be adequately monitored at reaction site **4**.

In operation, liquid sample is applied to port **2** and is drawn by capillary action through capillary space **3** to reaction site **4** where it is held by capillary forces. As the liquid sample moves through capillary spaces **3** and **4**, it will resolubilize reagent layers **6** and **7** so that magnetizable particles held in layer **6** are free to oscillate in response to an oscillating magnetic field and so that the thromboplastin held in reagent layer **7** begins to cause the specimen to coagulate. The oscillation of the magnetizable particles can then be monitored to determine clotting time as is described more fully in U.S. Patents No. 4,849,340, and 5,110,727.

FIG. 2 shows in enlarged schematic cross section device **1** of FIG. 1 along lines 2-2 of FIG. 1. Reagent layer **6** is coated onto the top surface **8** of device **1**. Layer **6** contains magnetizable particles **9** suspended in a dried mixture of carbohydrates of both high and low molecular weights **10**. Layer **7** in this particular embodiment is coated on layer **6**. As described above, layer **7** includes a mixture of both high and low carbohydrates with thromboplastin in an amount sufficient to begin a coagulation cascade in a blood or serum specimen which is drawn into the capillary space at reaction site **4**.

Although not shown, it will be apparent to the skilled artisan that layer **7** could also be positioned on lower surface **11** of capillary space **4** or even between layer **6** and surface **8**.

FIG. 3 shows in schematic cross section an alternative embodiment of the device of the present invention. In this embodiment the thromboplastin and the magnetizable particles **9** are suspended together in a single reagent layer **12** which is coated onto top surface **8** of device **1**.

Although the embodiment of FIG. 3 is within the scope of the present invention and has been shown to be useful, it is not preferred for two major reasons. One is that coating of a reagent layer including thromboplastin across the total capillary space (**3** and **4** in FIG. 1) can result in undesirably early activation of the coagulation cascade in the specimen before the specimen has reached the assay site **4**. Monitoring of the oscillation of the particles gives the most accurate results when the oscillation is observed from the beginning of the coagulation event.

A second reason that the embodiment of FIG. 2 is preferred is that the reagent layers may be applied sequentially and dried at different temperatures, ultimately requiring less of the thromboplastin material. The thromboplastin reagent is fragile at high temperatures, which has been one motivation in the past to freeze dry reagent materials for use in coagulation assays. Air drying a reagent layer such as that shown in FIG. 3 requires relatively high starting concentrations of thromboplastin because some of it loses its activity in the heat required for drying reagent layer **12**.

The assay device of Figs. 1 and 2 is made in a continuous process by first applying a spacer to a flexible plastic backing. The backing serves as surface **8** in FIGS. 2 and 3. The spacer contains a cutout which is substantially the shape of capillary spaces **3** and **4** in FIG. 1. The magnetic particle reagent suspension described above is dispensed into the cutout and air dried with heat at about 65° C until dried. In an average size cutout for use in coagulation assay meters about 30 microliters of the magnetic particle containing suspension is dispensed and dried into layer **6**. Subsequently, a small amount, normally about 2 microliters, of thromboplastin containing solution is dispensed on the top of layer **6** and air dried for about half the total drying time of first layer **6** at temperatures below about 55° C. to form layer **7**. After drying layer **6** and **7** normally have a combined thickness of between 10 and 20 microns. After the thromboplastin solution has been dispensed and dried openings **2** and **5** are formed in the back and a cover is placed over the spacer to form a capillary space containing the reagent layers.

The embodiment of FIG. 3 has been manufactured in a similar manner but with only one dispensing step for the combined solution of ingredients and a single drying step.

It will be apparent to the skilled artisan that the manufacturing process may include aids such as surfactants and corona treatments to encourage coating of the reagent on the surface.

### Examples

### Example 1

Layer 6 of FIG. 2 was made as follows: In 92 g. of water dissolve 4 g. each of sucrose and agglomerated corn syrup solids (M-600). In the resulting solution suspend finely particulated Fe₃O₄ by vigorous mixing. Vigorous mixing was maintained as 30 microliters of this suspension was dispensed into a preformed cup. The dispensed suspension was dried at 65° C for about 10 minutes.

### Example 2

Layer 7 of Fig. 2 was made as follows: 0.5 g. of thromboplastin (as total protien) from lyophylized powder was mixed with 100 g. of water. 2.96 g. each of sucrose and agglomerated corn syrup solids (M-600) were dissolved in the solution by mixing. About 2 microliters of this solution was dispensed on layer 6 formed in Example 1 and dried at about 55° C. for 5 minutes.

### Example 3

The structure formed by the procedures of Examples 1 and 2 was placed in an oscillating magnetic field and contacted with 10 microliters of a whole blood specimen from a pipette and observed to determine how quickly after contact by the liquid specimen the magnetizable particles began to oscillate in response to the oscillating magnetic field. The particles were observed to begin oscillation substantially simultaneously upon contact by the liquid specimen (within less than 0.5 second).

The present invention has been disclosed in the above teachings and drawing with sufficient clarity and conciseness to enable one skilled in the art to make and use the invention, to know the best mode for carrying out the invention and to distinguish it from other inventions and from what is old.

## Claims

1. A coatable dry reagent composition comprising:
(a) thromboplastin
(b) magnetizable particles; and
(c) a carrier which comprises a mixture of soluble carbohydrates of at least two different molecular weights, comprising pentasaccharides and above as higher molecular weight carbohydrates; and dissacharides as lower molecular weight carbohydrates.

2. The reagent of claim 1 wherein the disaccharides are sucrose and the pentasaccharides and above are provided by agglomerated corn syrup solids.

3. The reagent of claim 2 wherein the weight ratio of sucrose : agglomerated corn syrup solids : magnetizable particles : thromboplastin is within the range of 105-160 : 105-160 : 12-24 : 0.64 - 1.2.

4. The reagent of claim 3 wherein the weight ratio of sucrose : agglomerated corn syrup solids : magnetizable particles : thromboplastin is 120 : 120: 18 : 1.

5. A coatable dry reagent system comprising the reagent of claim 1 in which
(a) a first dry reagent layer comprises components (b) and (c) according to claim 1; and
(b) a second dry reagent layer comprises components (a) and (c) according to claim 1.

6. The reagent system of claim 5 wherein the disaccharides are sucrose and the pentasaccharides and above are provided by agglomerated corn syrup solids.

7. The reagent system of claim 6 wherein in the first layer the weight ratio of sucrose : agglomerated corn syrup solids : magnetizable particles is within the range 6-9 : 6-9 : 0.66-1.33.

8. The reagent system of claim 7 wherein the weight ratio of sucrose : agglomerated corn syrup solids : magnetizable particles is about 6.66 : 6.66 : 1.

9. The reagent system of claim 6 wherein in the second layer the weight ratio of sucrose : agglomerated corn syrup solids : thromboplastin is within the range 4-9: 4-9: 0.64-1.22.

10. The reagent system of claim 9 wherein the weight ratio of sucrose: agglomerated corn syrup solids : thromboplastin is about 6 : 6 : 1.

11. A method for performing a coagulation time assay on a whole blood or plasma specimen, comprising:
(a) contacting a whole blood or plasma specimen with the dry reagent composition of claim 1 or 5, whereby the composition is solubilized by the sample to form a reaction solution;
(b) subjecting the reaction solution to an oscillating magnetic field; and
(c) monitoring the oscillation of the magnetizable particles in the reaction solution in response to the oscillating magnetic field as an indication of the coagulation time of the specimen.

12. The method of claim 11 wherein the composition is coated onto the reaction surface in a capillary space which transports and holds the specimen in contact with the composition.

## Patentansprüche

1. Schichtbildende, trockene Reagenzzusammensetzung, die umfaßt:
(a) Thromboplastin
(b) magnetisierbare Teilchen und
(c) einen Träger, der eine Mischung aus löslichen Kohlenhydraten mit mindestens zwei verschiedenen Molekulargewichten umfaßt, die Pentasaccharide und höhere als Kohlenhydrate mit höherem Molekulargewicht und Disaccharide als Kohlenhydrate mit niedrigerem Molekulargewicht umfaßt.

2. Reagenz nach Anspruch 1, worin die Disaccharide Saccharose sind und die Pentasaccharide und höheren durch agglomerierte Maissirupfeststoffe zur Verfügung gestellt sind.

3. Reagenz nach Anspruch 2, worin das Gewichtsverhältnis von Saccharose : agglomerierten Maissirupfeststoffen : magnetisierbaren Teilchen : Thromboplastin innerhalb eines Bereichs von 105-160 : 105-160 : 12-24 : 0,64 - 1,2 liegt.

4. Reagenz nach Anspruch 3, worin das Gewichtsverhältnis von Saccharose : agglomerierten Maissirupfeststoffen : magnetisierbaren Teilchen : Thromboplastin 120 : 120 : 18 : 1 beträgt.

5. Schichtbildendes, trockenes Reagenzsystem, das das Reagenz von Anspruch 1 umfaßt, worin
(a) eine erste trockene Reagenzschicht die Komponenten (b) und (c) nach Anspruch 1 enthält und
(b) eine zweite trockene Reagenzschicht die Komponenten (a) und (c) nach Anspruch 1 enthält.

6. Reagenzsystein nach Anspruch 5, worin die Disaccharide Saccharose sind und die Pentasaccharide und höheren durch agglomerierte Maissirupfeststoffe zur Verfügung gestellt sind.

7. Reagenzsystem nach Anspruch 6, worin in der ersten Schicht das Gewichtsverhältnis von Saccharose : agglomerierten Maissirupfeststoffen : magnetisierbaren Teilchen innerhalb eines Bereichs von 6-9 : 6-9 : 0,66 - 1,33 liegt.

8. Reagenzsystem nach Anspruch 7, worin das Gewichtsverhältnis von Saccharose: agglomerierten Maissirupfeststoffen: magnetisierbaren Teilchen etwa 6,66 : 6,66 : 1 beträgt.

9. Reagenzsystem nach Anspruch 6, worin in der zweiten Schicht das Gewichtsverhältnis von Saccharose : agglomerierten Maissirupfeststoffen : Thromboplastin innerhalb eines Bereichs von 4-9 : 4-9 : 0,64-1,22 liegt.

10. Reagenzsystem nach Anspruch 9, worin das Gewichtsverhältnis von Saccharose: agglomerierten Maissirupfeststoffen: Thromboplastin etwa 6 : 6 : 1 beträgt.

11. Verfahren zur Durchführung eines Koagulationszeit-Assays mit einer Gesamtblutprobe oder Plasmaprobe, das aufweist:
(a) in Kontakt bringen einer Gesamtblutprobe oder einer Plasmaprobe mit der trockenen Reagenzzusammensetzung von Anspruch 1 oder 5, wobei die Zusammensetzung. durch die Probe solubilisiert wird und eine Reaktionslösung bildet;
(b) Unterwerfen der Reaktionslösung einem oszillierenden magnetischen Feld und
(c) Überwachen der Oszillierung der magnetisierbaren Teilchen in der Reaktionslösung als Antwort auf das oszillierende magnetische Feld als Indikation für die Koagulationszeit der Probe.

12. Verfahren nach Anspruch 11, worin die Zusammensetzung auf die Reaktionsoberfläche in einem Kapillarraum, der die Probe in Kontakt mit der Zusammensetzung transportiert und hält, aufgetragen wird.

## Revendications

1. Composition de réactif sec applicable comprenant :
(a) de la thromboplastine ;
(b) des particules magnétisables ; et
(c) un support qui comprend un mélange de glucides solubles d'au moins deux poids moléculaires différents, comprenant
des pentasaccharides et saccharides supérieurs en tant que glucides de poids moléculaire supérieur ; et
des disaccharides en tant que glucides de poids moléculaire inférieur.

2. Réactif selon la revendication 1 dans lequel les disaccharides sont du saccharose et les pentasaccharides et saccharides supérieurs sont apportés par du sirop de glucose déshydraté aggloméré.

3. Réactif selon la revendication 2 dans lequel le rapport pondéral du saccharose:sirop de glucose déshydraté aggloméré:particules magnétisables:thromboplastine se situe dans la gamme de 105-160:105-160:12-24:0,64-1,2.

4. Réactif selon la revendication 3 dans lequel le rapport pondéral du saccharose:sirop de glucose déshydraté aggloméré:particules magnétisables:thromboplastine est de 120:120:18:1.

5. Système de réactif sec applicable comprenant le réactif selon la revendication 1 dans lequel
(a) une première couche de réactif sec comprend les composants (b) et (c) selon la revendication 1 ;
(b) une seconde couche de réactif sec comprend les composants (a) et (c) selon la revendication 1.

6. Système de réactif selon la revendication 5 dans lequel les dissacharides sont du saccharose et les pentasaccharides et saccharides supérieurs sont apportés par du sirop de glucose déshydraté aggloméré.

7. Système de réactif selon la revendication 6 dans lequel dans la première couche le rapport pondéral du saccharose:sirop de glucose déshydraté aggloméré:particules magnétisables se situe dans la gamme de 6-9:6-9:0,66-1,33.

8. Système de réactif selon la revendication 7 dans lequel le rapport pondéral du saccharose:sirop de glucose déshydraté aggloméré:particules magnétisables est d'environ 6,66:6,66:1.

9. Système de réactif selon la revendication 6 dans lequel dans la seconde couche le rapport pondéral du saccharose:sirop de glucose déshydraté aggloméré:thromboplastine se situe dans la gamme de 4-9:4-9:0,64-1,22.

10. Système de réactif selon la revendication 9 dans lequel le rapport pondéral du saccharose:sirop de glucose déshydraté aggloméré:thromboplastine est d'environ 6:6:1.

11. Procédé de réalisation d'une mesure de temps de coagulation sur un échantillon de sang complet ou de plasma, consistant à :
(a) mettre en contact un échantillon de sang complet ou de plasma avec la composition de réactif sec selon la revendication 1 ou 5, grâce à quoi la composition est solubilisée par l'échantillon pour former une solution réactionnelle ;
(b) exposer la solution réactionnelle à un champ magnétique oscillant ; et
(c) contrôler l'oscillation des particules magnétisables dans la solution réactionnelle en réponse au champ magnétique oscillant en tant qu'indication du temps de coagulation de l'échantillon.

12. Procédé selon la revendication 11 dans lequel la composition est appliquée sur la surface réactionnelle dans un espace capillaire qui transporte et maintient l'échantillon en contact avec la composition.
